# EUROPÄISCHE PATENTSCHRIFT

(11) **EP 2 559 377 B1**
(45) Veröffentlichungstag und Bekanntmachung des Hinweises auf die Patenterteilung: **11.09.2019**
(21) Anmeldenummer: 12178482.1
(22) Anmeldetag: 30.07.2012
(51) Int. Cl.: A61B 5/0215, A61B 5/08

(54) **VERFAHREN UND IMPLANTIERBARES MEDIZINISCHES SYSTEM ZUM MONITORING VON ATEMPARAMETERN SOWIE EIN ENTSPRECHENDES COMPUTERPROGRAMM UND EIN ENTSPRECHENDES COMPUTERLESBARES SPEICHERMEDIUM**
METHOD AND IMPLANTABLE MEDICAL SYSTEM FOR MONITORING RESPIRATORY PARAMETERS AND A CORRESPONDING COMPUTER PROGRAM AND A CORRESPONDING COMPUTER-READABLE STORAGE MEDIUM
PROCÉDÉ ET SYSTÈME MÉDICAL IMPLANTABLE DESTINÉ À LA SURVEILLANCE DE PARAMÈTRES RESPIRATOIRES AINSI QU'UN PROGRAMME INFORMATIQUE CORRESPONDANT ET UN SUPPORT DE STOCKAGE LISIBLE SUR ORDINATEUR CORRESPONDANT

(30) Priorität: 17.08.2011 US 201161524351 P
(43) Veröffentlichungstag der Anmeldung: 20.02.2013
(73) Patentinhaber: BIOTRONIK SE & Co. KG, 12359 Berlin (DE)
(72) Erfinder: KIRCHNER, Jens, 91052 Erlangen (DE)
(74) Vertreter: Galander, Marcus

(56) Entgegenhaltungen:
- EP-A2- 2 008 581
- EP-A2- 2 030 564
- US-B1- 6 350 242

## Beschreibung

Die Erfindung betrifft ein Verfahren und ein implantierbares medizinisches System zum Monitoring von Atemparametern sowie ein entsprechendes Computerprogramm und ein entsprechendes computerlesbares Speichermedium, welche insbesondere einsetzbar sind zur Überwachung, speziell zur Fernüberwachung, des Gesundheitszustandes von Patienten mit Herzinsuffizienz.

Auf dem Gebiet der Erfindung sind bereits Lösungen bekannt, bei denen zur Überwachung des Gesundheitszustandes Impedanz- und Blutdrucksignale ausgewertet werden. Nachteilig an den bisherigen Lösungen ist insbesondere, dass bei den bekannten Verfahren der Belastungszustand eines Patienten nicht berücksichtigt wird.

So beschreibt die EP2008581 ausführlich verschiedene Möglichkeiten zum Monitoren von Gesundheitszuständen mittels einer Vielzahl von Möglichen Parametern.

Die US6350242 bezieht sich auf die Analyse von Parametern, insbesondere des Blutdruckes, um aus diesen die Atemrate zu extrapolieren.

Es ist daher Aufgabe der Erfindung, ein Verfahren und ein implantierbares medizinisches System zum Monitoring von Atemparametern sowie ein entsprechende Computerprogramm und ein entsprechendes computerlesbares Speichermedium bereitzustellen, welche die Nachteile der herkömmlichen Lösungen vermeiden und insbesondere ein verbessertes Verfahren zur Bestimmung der Leistungsfähigkeit des Herz-Kreislauf-Systems unter Berücksichtigung der Belastung bereitstellen.

Die Aufgabe wird durch ein medizinisches System nach Anspruch 1, sowie durch das Verfahren nach Anspruch 8 durch ein Computerprogramm nach Anspruch 10 und ein computerlesbares Speichermedium nach Anspruch 11 gelöst.

Das implantierbare medizinische System umfasst mindestens einen Drucksensor, eine Zeiterfassungseinheit und eine Datenverarbeitungseinheit, wobei das System derart eingerichtet ist, dass folgende Schritte ausführbar sind:
- zumindest zeitweiliges Erfassen von Blutdruckwerten,
- durch Auswertung der erfassten Blutdruckwerte Bestimmen von Daten, die eine Belastung beschreiben,
- durch Auswertung der erfassten Blutdruckwerte Bestimmen von Daten, die die Atmung beschreiben,
- Auswertung der Atmung zur Bestimmung einer diagnostischen Größe in Abhängigkeit von den die Belastung beschreibenden Daten.

Die "Auswertung der Atmung zur Bestimmung einer diagnostischen Größe in Abhängigkeit von den die Belastung beschreibenden Daten" bezieht sich sowohl auf die Abhängigkeit der Atmung von den die Belastung beschreibenden Daten, wie auch die Abhängigkeit von den die Belastung beschreibenden Daten von der Atmung.

Ein besonderes Kennzeichen des erfindungsgemäßen Verfahrens besteht u.a. darin, dass neben Parametern, die herkömmlicherweise bei der Überwachung von Herzinsuffizienz ausgewertet werden, zusätzlich die Belastung des Patienten erfasst und ausgewertet wird. Dabei bietet das erfindungsgemäße Verfahren den besonderen Vorteil, dass dies ohne Verwendung zusätzlicher Geräte, wie z.B. Referenzsensoren, erreicht wird. Das erfindungsgemäße Verfahren sieht daher vor, dass zum Monitoring der Atemparameter lediglich eine Blutdruckmessung durchgeführt wird. Dazu wird mindestens ein Drucksensor genutzt, wobei der mindestens eine Drucksensor vorzugsweise in der Pulmonalarterie und/oder der Aorta und/oder den peripheren Arterien und/oder den peripheren Venen und/oder den herznahe Venen und/oder den Herzkammern angeordnet ist. Von dem mindestens einen Drucksensor werden zumindest zeitweise, vorzugsweise aber kontinuierlich, Signale erfasst und durch Auswertung der erfassten Signale Blutdruckwerte bzw. Werte, die mit dem Blutdruck in Zusammenhang stehen, bestimmt. Zumindest zur Bestimmung der Blutdruckwerte wird weiter mindestens eine Datenverarbeitungseinheit eingesetzt. Vorzugsweise wird die mindestens eine Datenverarbeitungseinheit auch genutzt, um aus den erfassten Signalen oder den bestimmten Blutdruckwerten Daten (Belastungswerte) zu ermitteln, welche eine Belastung, insbesondere eine physische Belastung, des Patienten beschreiben bzw. mit der Belastung in Zusammenhang stehen. Beispielsweise kann aus den durch den mindestens einen Drucksensor erfassten Signalen die Herzrate extrahiert werden, die als eine Beschreibung der Belastung bzw. der Aktivität des Patienten genutzt wird. Weiter werden erfindungsgemäß aus den erfassten Signalen oder den bestimmten Blutdruckwerten Daten ermittelt, welche die Atmung beschreiben bzw. mit der Atmung in Zusammenhang stehen. In einer bevorzugten Ausführungsform geschieht dies, indem charakteristische Punkte des Blutdrucksignals ausgewertet werden. Bei diesen charakteristischen Punkten kann es sich beispielsweise um zumindest eins der folgenden Merkmale handeln:
- Beginn einer Systole,
- Ende einer Systole,
- Maximaldruck innerhalb eines Herzzyklus,
- Minimaldruck innerhalb eines Herzzyklus,
- Extremalwerte der ersten Ableitung des Blutdrucks.

Aus den charakteristischen Punkten, wie z.B. den Startzeitpunkten der Systolen oder den Druckmaxima der Zyklen, wird das Atemsignal ermittelt. Dies ist insbesondere deshalb möglich, weil die Werte des Blutdruckes eine Überlagerung der Blutdruckvariationen des Atemzyklus und der der Herzkontraktion darstellen. Die charakteristischen Punkte können daher als eine gute Näherung für die Atmung angesehen werden. In einer bevorzugten Ausführungsform werden zur Ermittlung der Daten, die die Atmung beschreiben, Schwellwerte verwendet.

Erfindungsgemäß wird aus den Daten, die mit der Atmung in Zusammenhang stehen, mindestens eine Kenngröße ermittelt. Bei dieser mindestens einen Kenngröße kann es sich beispielsweise um eins der folgenden Merkmale des Atemsignals handeln:
- Frequenz,
- Frequenzvariabilität,
- Amplitude,
- maximale Steigung,
- minimale Steigung,
- Anzahl der Peaks pro Atemzyklus,
- Anzahl der Plateaus pro Atemzyklus,
- Dauer des Anstiegs,
- Dauer des Abfalls,
- Dauer der Pause zwischen zwei Zyklen, d.h. die Zeit, in der keine Änderung des Thoraxdrucks festgestellt wird,
- Integral eines Atemzyklus.

Erfindungsgemäß wird aus der mindestens einen Kenngröße erfindungsgemäß mindestens eine diagnostische Größe abgeleitet, welche die Abhängigkeit der Atmung von dem Belastungswert beschreibt. Vorzugsweise umfasst die mindestens eine diagnostische Größe Parameter einer Kurve der mindestens einen Kenngröße in Abhängigkeit von der Belastung. Die mindestens eine diagnostische Größe kann z.B. mindestens einen der folgenden Parameter umfassen:
- mittlere, minimale und/oder maximale Steigung der Kurve;
- mittlere, minimale und/oder maximale Steigung der Kurve in einem definierten Belastungsbereich;
- Wert der Belastung an einem Abknickpunkt, ab dem die Kurve konstant verläuft;
- Fitparameter einer definierten Fitfunktion;
- Ort und Breite eines Belastungsbereichs, in dem die Kurve eine definierte funktionale Abhängigkeit zeigt.

In einer bevorzugten Ausführungsform der Erfindung ist vorgesehen, dass die Daten, die mit der Atmung in Zusammenhang stehen, insbesondere die mindestens eine Kenngröße, die für verschiedene Belastungen ermittelt wurden, miteinander verglichen werden.

In einem Beispiel ist vorgesehen, dass der Wertebereich der Daten, die die Belastung beschreiben, in Intervalle (Belastungsintervalle) einer vorgebbaren Länge unterteilt wird. Eine bevorzugte Ausführungsform sieht vor, für diese Intervalle eine variable Länge vorzugeben. Alternativ kann auch stets dieselbe Länge vorgegeben werden. Vorzugsweise wird zumindest einem Teil der Intervalle eine Belastungsstufe zugewiesen. Die zugewiesene Belastungsstufe kann beispielsweise einem definierten Wertebereich entnommen werden, z.B. die mittlere Belastung eines Belastungsintervalls, oder, wenn es sich um diskrete Belastungswerte handelt, kann die zugewiesene Belastungsstufe einer definierten Wertemenge entnommen werden.

Erfindungsgemäß ist vorgesehen, dass in Anhängigkeit der Belastung Zeitintervalle bestimmt werden. Eine bevorzugte Ausführungsform sieht dabei vor, dass die Länge der Zeitintervalle so bestimmt wird, dass sich der Patient innerhalb eines Zeitintervalls ausschließlich auf einer Belastungsstufe befindet, d.h.: dass innerhalb eines Zeitintervalls ausschließlich solche eine Belastung beschreibenden Daten (Belastungsdaten) erfasst wurden, die in ein und demselben Belastungsintervall liegen. Anhand der den Belastungsintervallen zugewiesenen Belastungsstufen können nun Zeitintervalle ausgewertet werden, die vorgegebene Belastungswerte umfassen. Insbesondere können Daten ausgewertet werden, die mit der Atmung in Zusammenhang stehen. Eine beispielhafte Auswertung kann darin bestehen, dass für eine Menge von Zeitintervallen der Mittelwert zumindest eines Teils dieser mit der Atmung in Zusammenhang stehenden Daten gebildet wird. Eine beispielhafte Ausführungsform sieht vor, dass die Auswertung alle Zeitintervalle eines Tages erfasst, welche Belastungswerte einer vorgebbaren Belastungsstufe umfassen. Beispielsweise kann für jede Belastungsstufe ein Tagesmittelwert für die Atemfrequenz gebildet werden.

Die Zeitintervalle sind mit der Zeiterfassungseinheit bestimmbar.

Um die Zeitintervalle zu bestimmen, wird vorzugsweise die Änderung der Belastung überwacht. Durch Auswertung der Änderung der Belastung werden Zeitpunkte detektiert, zu denen die Belastung von einem Belastungsintervall zu einem anderen übergeht. Die detektierten Zeitpunkte definieren die Zeitintervalle. Dadurch wird ermöglicht, dass bei einer Auswertung der Atemparameter ausschließlich solche Intervalle berücksichtigt werden, in denen die Belastung für eine definierte Zeit einen Wertebereich nicht verlässt. Somit können Atemparameter in Abhängigkeit einer Belastungsstufe ausgewertet werden.

In einer weiteren bevorzugten Ausführungsform ist vorgesehen, dass die mindestens eine diagnostische Größe aus der Kurve durch Fitten abgeleitet wird. Insbesondere können beispielsweise Fitparameter, wie z.B. Steigung und/oder Offset, Zeitkonstanten o.ä., ausgewertet werden. Alternativ ist vorgesehen, dass mindestens eine diagnostische Größe aus der Kurve gewonnen wird, indem mindestens zwei Fitfunktionen an einen vorgebbaren Bereich der Kurve, welche das Verhalten der Kenngröße der Atmung in Abhängigkeit von der Belastung beschreibt, gefittet werden und die Güte des Fits und/oder Fitparameter bestimmt und ausgewertet werden. Insbesondere können beispielsweise der Typ der optimalen Fitfunktion und/oder eine aus einem Vektor der Fitgüte abgeleitete Größe ausgewertet werden.

Das Fitten einer Fitfunktion zur Bestimmung eines optimalen Fitparameters einerseits und der Test, welche Fitfunktion aus einer Menge von Templates am besten passt, andererseits sind zwei unterschiedliche Ansätze. Beim Fitten einer einzigen Funktion wird eine gewisse Form (Fitfunktion) vorausgesetzt und von Interesse sind die besonderen Ausprägung, d.h. die Fitparameter. Beim Testen verschiedener Funktionen sind die zu erwartenden Formen unbekannt und es werden verschiedene Möglichkeiten getestet. Das Ergebnis ist dann eine Entscheidung über die am besten passende Form. Die zugehörigen Fitparameter sind in der Regel weniger interessant. Vor allem kann ein freier Parameter einer bestimmten Fitfunktion bei einer anderen Fitfunktion in einem völlig anderen Wertebereich liegen oder sogar gar nicht definiert oder sinnvoll sein.

Eine andere bevorzugte Ausführungsform sieht vor, dass die Belastungsdaten durch einen Aktivitätssensor erfasst werden.

Ein weiterer Aspekt der Erfindung besteht in einem implantierbaren System, welches mindestens einen Drucksensor, eine Zeiterfassungseinheit und mindestens eine Datenverarbeitungseinheit umfasst und derart eingerichtet ist, dass ein Verfahren zum Monitoring von Atemparametern ausführbar ist, wobei zumindest zeitweilig Blutdruckwerte erfasst werden, anhand der erfassten Blutdruckwerte Daten, die eine Belastung beschreiben, und Daten, die die Atmung beschreiben, bestimmt werden und mindestens eine diagnostische Größe aus der Auswertung der die Atmung beschreibenden Daten in Abhängigkeit von den die Belastung beschreibenden Daten bestimmt wird.

Eine andere bevorzugte Ausführungsform des implantierbaren Systems umfasst außerdem mindestens einen Aktivitätssensor und ist derart eingerichtet, dass ein Verfahren zum Monitoring von Atemparametern ausführbar ist, wobei zumindest zeitweilig Signalen des mindestens einen Aktivitätssensors erfasst werden und zumindest ein Belastungswert durch Auswertung der Signale des Aktivitätssensors bestimmt wird, und wobei weiter zumindest zeitweilig Blutdruckwerte erfasst werden, anhand der erfassten Blutdruckwerte Daten, die die Atmung beschreiben, bestimmt werden und mindestens eine Größe aus der Auswertung der die Atmung beschreibenden Daten in Abhängigkeit von den die Belastung beschreibenden Daten bestimmt wird.

Eine bevorzugte Ausführungsform des implantierbaren Systems sieht vor, dass der mindestens eine Drucksensor vorzugsweise in der der Pulmonalarterie und/oder der Aorta und/oder den peripheren Arterien und/oder den peripheren Venen und/oder den herznahe Venen und/oder den Herzkammern angeordnet ist.

### Im übrigen sind zu jeder der beschriebenen besonderen Ausführungsformen des erfindungsgemäßen Verfahrens auch die entsprechenden implantierbaren Systeme vorgesehen

Um das Blutdrucksignal zu Zeiten einer definierten Belastung auszuwerten, ist in einer bevorzugten Ausführungsform ein implantierbares System vorgesehen, welches mindestens
- einen Aktivitätssensor,
- einen Blutdrucksensor und
- eine Datenverarbeitungseinheit als Auswerteeinheit,
umfasst und darauf ausgelegt ist, die folgenden Aufgaben zu erfüllen:
a) Messung eines Signals, das in Zusammenhang mit der physischen Belastung steht,
b) Messung eines Signals, das in Zusammenhang mit einem Blutdruck steht,
c) Bestimmung von zumindest einem Zeitintervall zur Signalauswertung unter Verwendung des Belastungssignals,
d) Ausführung der folgenden Schritte für jedes der in c) bestimmten Zeitintervalle:
   (i) Bestimmung von charakteristischen Punkten im Blutdrucksignal,
   (ii) Bestimmung eines Signals aus den in Schritt (i) bestimmten Punkten, welches in Zusammenhang mit der Atmung steht,
   (iii) Bestimmung einer Kenngröße aus dem in Schritt (ii) bestimmten Signal,
e) Bestimmung einer diagnostischen Größe aus der Menge der in Schritt d) bestimmten Werte, beispielsweise durch Mittelung.

Um die Kenngröße bei unterschiedlichen Belastungen zu vergleichen, ist in einer bevorzugten Ausführungsform ein implantierbares System vorgesehen, welches mindestens
- einen Aktivitätssensor,
- einen Blutdrucksensor und
- eine Datenverarbeitungseinheit als Auswerteeinheit,
umfasst und darauf ausgelegt ist, die folgenden Aufgaben zu erfüllen:
a) Messung eines Signals, das in Zusammenhang mit der physischen Belastung steht,
b) Messung eines Signals, das in Zusammenhang mit einem Blutdruck steht,
c) Bestimmung von mehreren Zeitintervallen zur Signalauswertung, evtl. unter Verwendung des Belastungssignals,
d) Ausführung der folgenden Schritte für jedes der in c) bestimmten Zeitintervalle:
   (i) Zuweisung eines Belastungswertes zu dem Zeitintervall,
   (ii) Bestimmung von charakteristischen Punkten im Blutdrucksignal,
   (iii) Bestimmung eines Signals aus den in Schritt (ii) bestimmten Punkten, welches in Zusammenhang mit der Atmung steht,
   (iv) Bestimmung einer Kenngröße aus dem in Schritt (iii) bestimmten Signal,
e) Bestimmung einer diagnostischen Größe aus den in Schritt d) erzeugten Datenpaaren aus Belastung (Schritt (i)) und Kenngröße der Atmung (Schritt (iv)).

Ein Computerprogramm nach der Erfindung ermöglicht es einer Datenverarbeitungseinrichtung, nachdem es in Speichermittel der Datenverarbeitungseinrichtung geladen worden ist, ein Verfahren zum Monitoring von Atemparametern durchzuführen, wobei
- zumindest zeitweilig Blutdruckwerte erfasst werden,
- durch Auswertung der erfassten Blutdruckwerte Daten bestimmt werden, die eine Belastung beschreiben,
- durch Auswertung der erfassten Blutdruckwerte Daten bestimmt werden, die die Atmung beschreiben,
- eine Abhängigkeit der Atmung von den die Belastung beschreibenden Daten oder die eine Abhängigkeit der die Belastung beschreibenden Daten von der Atmung ausgewertet wird zur Bestimmung einer diagnostischen Größe.

Ein anderes Computerprogramm nach der Erfindung ermöglicht es einer Datenverarbeitungseinrichtung, nachdem es in Speichermittel der Datenverarbeitungseinrichtung geladen worden ist, ein alternatives Verfahren zum Monitoring von Atemparametern durchzuführen, wobei
- zumindest zeitweilig Signale mindestens eines Aktivitätssensors erfasst und durch Auswertung der Signale des mindestens einen Aktivitätssensors Daten bestimmt werden, die eine Belastung beschreiben,
- zumindest zeitweilig Blutdruckwerte erfasst werden,
- durch Auswertung der erfassten Blutdruckwerte Daten bestimmt werden, die die Atmung beschreiben,
- eine Abhängigkeit der Atmung von den die Belastung beschreibenden Daten oder die eine Abhängigkeit der die Belastung beschreibenden Daten von der Atmung ausgewertet wird zur Bestimmung einer diagnostischen Größe.

In einer weiteren bevorzugten Ausführungsform der Erfindung ist vorgesehen, dass das erfindungsgemäße Computerprogramm modular aufgebaut ist, wobei einzelne Module auf verschiedenen Teilen der Datenverarbeitungseinrichtung installiert sind.

Vorteilhafte Ausführungsformen sehen zusätzlich Computerprogramme vor, durch welche weitere in der Beschreibung angegebene Verfahrensschritte oder Verfahrensabläufe ausgeführt werden können.

Solche Computerprogramme können beispielsweise (gegen Gebühr oder unentgeltlich, frei zugänglich oder passwortgeschützt) downloadbar in einem Daten- oder Kommunikationsnetz bereitgestellt werden. Die so bereitgestellten Computerprogramme können dann durch ein Verfahren nutzbar gemacht werden, bei dem ein Computerprogramm aus einem elektronischen Datennetz, wie beispielsweise aus dem Internet, auf eine an das Datennetz angeschlossene Datenverarbeitungseinrichtung heruntergeladen wird.

Um das erfindungsgemäße Verfahren zum Monitoring von Atemparametern durchzuführen, ist vorgesehen, ein computerlesbares Speichermedium einzusetzen, auf dem ein Programm gespeichert ist, das es einer Datenverarbeitungseinrichtung ermöglicht, nachdem es in Speichermittel der Datenverarbeitungseinrichtung geladen worden ist, ein Verfahren zum Monitoring von Atemparametern durchzuführen, wobei
- zumindest zeitweilig Blutdruckwerte erfasst werden,
- durch Auswertung der erfassten Blutdruckwerte Daten bestimmt werden, die eine Belastung beschreiben,
- durch Auswertung der erfassten Blutdruckwerte Daten bestimmt werden, die die Atmung beschreiben,
- eine Abhängigkeit der Atmung von den die Belastung beschreibenden Daten oder die eine Abhängigkeit der die Belastung beschreibenden Daten von der Atmung ausgewertet wird zur Bestimmung einer diagnostischen Größe.

Es ist ebenfall erfindungsgemäß ein computerlesbares Speichermedium vorgesehen, auf dem ein Programm gespeichert ist, das es einer Datenverarbeitungseinrichtung ermöglicht, nachdem es in Speichermittel der Datenverarbeitungseinrichtung geladen worden ist, ein Verfahren zum Monitoring von Atemparametern durchzuführen, wobei
- zumindest zeitweilig Signale mindestens eines Aktivitätssensors erfasst und durch Auswertung der Signale des mindestens einen Aktivitätssensors Daten bestimmt werden, die eine Belastung beschreiben,
- zumindest zeitweilig Blutdruckwerte erfasst werden,
- durch Auswertung der erfassten Blutdruckwerte Daten bestimmt werden, die die Atmung beschreiben,
- eine Abhängigkeit der Atmung von den die Belastung beschreibenden Daten oder die eine Abhängigkeit der die Belastung beschreibenden Daten von der Atmung ausgewertet wird zur Bestimmung einer diagnostischen Größe.

Die Erfindung ist besonders vorteilhaft einsetzbar zum Monitoring von Herzinsuffizienz über Kurzatmigkeit.

Mit der Verschlechterung des Gesundheitszustandes bei Herzinsuffizienz sinkt die Leistungsfähigkeit des Herz-Kreislauf-Systems bei Belastung. Bei gegebener Belastung lässt sich die Leistungsfähigkeit an der Atmung (insbesondere Frequenz und Amplitude, aber auch Form des Atemzyklus') ablesen. Das hier vorgestellte System besteht aus einem Aktivitätssensor, der die Belastung des Patienten quantifiziert, sowie einem Blutdrucksensor, vorzugsweise in der Pulmonalarterie und/oder der Aorta und/oder den peripheren Arterien und/oder den peripheren Venen und/oder den herznahe Venen und/oder den Herzkammern, aus dessen Signal ein Atemsignal extrahiert wird. Die Auswertung von Kenngrößen dieses Atemsignals in Abhängigkeit von der Belastung dient zur Bestimmung der Leistungsfähigkeit des Patienten und damit zur Überwachung des Schweregrades seiner Herzinsuffizienz.
- Figur 1:: Beispielausschnitt einer Aufzeichnung des pulmolarteriellen Drucks über der Zeit,
- Figur 2:: Beispielausschnitt einer Aufzeichnung des pulmolarteriellen Drucks über der Zeit mit Angabe von Schwellwerten und
- Figur 3:: schematischer Plot der Wertepaare mittlere Atemfrequenz - physische Belastung.

Nachfolgend soll die Erfindung an einer beispielhaften Ausführungsform in größerem Detail beschrieben werden, wobei die Erfindung nicht auf dieses Ausführungsbeispiel beschränkt ist, sondern auch Lösungen umfasst, solange diese Lösungen nur die Merkmale der unabhängigen Ansprüche realisieren.

Die beispielhafte Ausführungsform ist als implantierbares System realisiert, welches einen Blutdrucksensor und einen Aktivitätssensor umfasst. Der Blutdrucksensor ist dabei in der Pulmonalarterie angeordnet, der Aktivitätssensor ist als Akzelerometer ausgeführt.

In der beispielhaften Ausführungsform ist die Erfindung in ein implantierbares System zur Überwachung des Herzzeitvolumens integriert. Das System weist neben dem Blutdrucksensor und dem Aktivitätssensor einen Sender und eine Antenne zur Datenübertragung an externe Geräte auf.

Um das erfindungsgemäße Verfahren auszuführen, wird in der beispielhaften Ausführungsform der Wertebereich des Aktivitätssensors in mehrere Aktivitätsniveaus bzw. - stufen aufgeteilt. Es sind beispielsweise sieben Stufen ,Ruhe', ,gering', ,mittel', ,hoch' sowie drei Zwischenstufen vorgesehen. Die Einteilung des Wertebereichs kann aber auch in eine höhere oder geringere Zahl von Stufen aufgeteilt werden.

Es ist weiter vorgesehen, dass disjunkte Zeitintervalle bestimmt werden, in denen sich der Patient ausschließlich in einer Belastungsstufe befindet. In einer bevorzugten Ausführungsform ist vorgesehen, dass die Zeitintervalle eine definierte, vorgegebene Länge, z.B. 2 min, haben. Für jedes dieser 2-Minuten-Intervalle wird nun das Signal 100 des pulmolarteriellen Drucks (pulmonary artery pressure, PAP) ausgewertet.

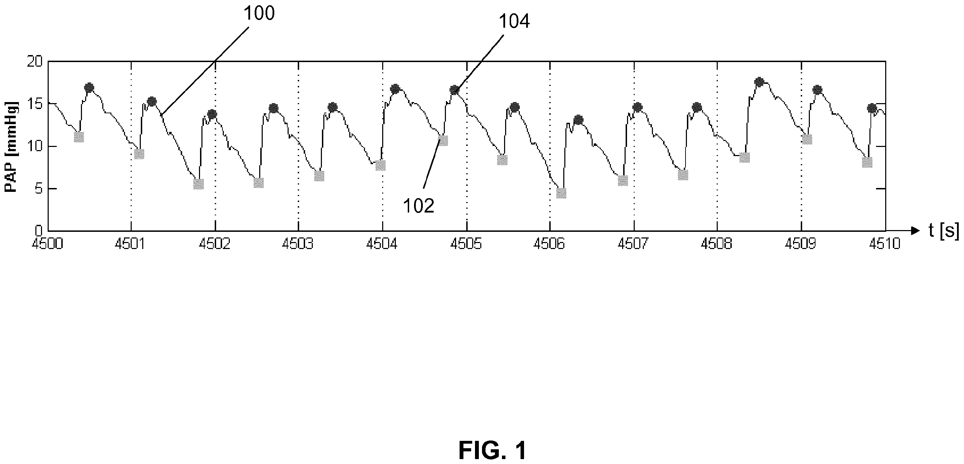

In Figur 1 ist ein Beispielausschnitt einer Aufzeichnung des PAP-Signals 100 über der Zeit dargestellt. Durch Auswertung des PAP-Signals 100 wird das Atemsignal rekonstruiert. Hierfür wird in dieser beispielhaften Ausführungsform als ein charakteristischer Punkt der Startzeitpunkt 102 der Systole jedes Zyklus ausgewertet. Alternativ dazu kann auch das Druckmaximum 104 jedes Zyklus ausgewertet werden. Die Druckwerte der Startzeitpunkte 102 über der Zeit stellen das rekonstruierte Atemsignal dar. Gegebenenfalls ist noch eine Baselineverschiebung erforderlich.

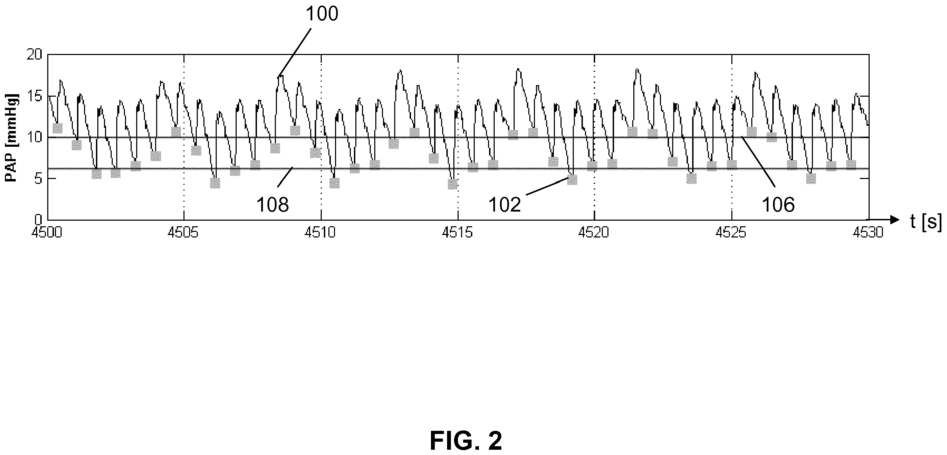

In dem rekonstruierten Atemsignal wird eine Zykluserkennung durchgeführt, wobei vorzugsweise ein Schwellenkriterium genutzt wird, wie aus Figur 2 an den beiden, Schwellwerte repräsentierenden horizontalen Linien 106, 108 verdeutlicht ist. Dabei wird in Figur 2 veranschaulicht, dass das Atemsignal aus dem Startzeitpunkt 102 der Systole jedes Zyklus ermittelt wird.

Aus Kenntnis des Atemzyklus werden nun Kenngrößen der Atmung, bspw. die Atemfrequenz, ermittelt. Um zu der mindestens einen diagnostischen Größe zu gelangen, wird in der beispielhaften Ausführungsform die Atemfrequenz über alle Intervalle derselben Belastungsstufe gemittelt. Diese Mittelung wird vorzugsweise für alle Belastungsstufen durchgeführt.

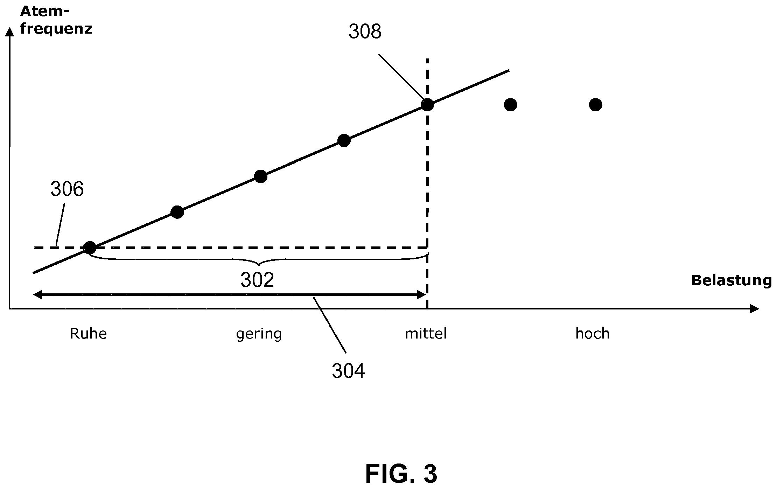

Aus dieser Mittelung resultieren Wertepaare (mittlere Atemfrequenz - Belastungsstufe), die als Funktion über der Belastung aufgetragen werden, wie in Figur 3 veranschaulicht ist. Figur 3 zeigt einen schematischen Plot 300 der Wertepaare (mittlere Atemfrequenz - Belastungsstufe), wobei die Belastung in sieben Stufen (,Ruhe', ,gering', ,mittel', ,hoch' sowie drei Zwischenstufen) unterteilt ist. Kenngrößen, die sich aus diesem Plot 300 ableiten lassen, sind beispielsweise die Steigung des Plots 300 im linear ansteigendem Bereich 302, Breite 304 des linear ansteigendem Bereichs 302, die Ruheatemfrequenz 306, der Abknickpunkt 308.

Wie erwähnt, ist in einer anderen beispielhaften Ausführungsform vorgesehen, dass das Belastungssignal aus dem PAP-Signal extrahiert wird. Der Einsatz eines Aktivitätssensors ist in dieser beispielhaften Ausführungsform nicht erforderlich. Vielmehr werden Herzrate als Aktivitätsmaß und Atemfrequenz oder -amplitude als Kenngröße der Atmung extrahiert.

In einer anderen alternativen Ausführungsform werden anstelle oder neben der Atemfrequenz andere Kenngrößen zur Beschreibung der Atmung verwendet. Als solche alternativen Kenngrößen können beispielsweise die Kurvensteilheit, die Anzahl von Peaks und/oder Plateaus innerhalb eines Atemzyklus', die Aufenthaltsdauer in einem bestimmten (relativen) Wertebereich (z.B. Dauer des Abfalls von 80 auf 20% des maximalen Differenzdrucks) betrachtet werden.

Die Funktion des Aktivitätssensors wird in einer weiteren alternativen Ausführungsform von einem Lagesensor übernommen, sodass verschiedene Körperlagen des Patienten als "unterschiedliche Belastungen" unterschieden werden können. Damit lässt sich insbesondere Orthopnoe, also die Luftnot im Liegen, detektieren.

Die Erfindung beschränkt sich in ihrer Ausführungsform nicht auf die vorstehend angegebenen bevorzugten Ausführungsbeispiele. Vielmehr ist eine Anzahl von Varianten denkbar, die von dem erfindungsgemäßen Verfahren, dem erfindungsgemäßen implantierbaren System, dem erfindungsgemäßen Computerprogramm oder dem erfindungsgemäßen computerlesbaren Speichermedium auch bei grundsätzlich anders gearteten Ausführungen Gebrauch machen.

## Patentansprüche

1. Implantierbares medizinisches System umfassend mindestens
- einen Drucksensor,
- eine Zeiterfassungseinheit und
- eine Datenverarbeitungseinheit,
wobei das System derart eingerichtet ist, dass folgende Schritte ausführbar sind:
- zumindest zeitweiliges Erfassen von Blutdruckwerten,
- durch Auswertung der erfassten Blutdruckwerte Bestimmen von Daten, die eine Belastung beschreiben,
- durch Auswertung der erfassten Blutdruckwerte Bestimmen von Daten, die die Atmung beschreiben,
**dadurch gekennzeichnet, dass** in Abhängigkeit der Belastung Zeitintervalle einer vorgebbaren Länge bestimmt werden, zumindest einem Teil der Zeitintervalle eine Belastungsstufe zugeordnet wird, und die die Atmung beschreibenden Daten für zumindest einen Teil der Zeitintervalle ausgewertet werden.

2. Implantierbares medizinisches System nach Anspruch 1, wobei aus den erfassten Blutdruckwerten die Herzrate bestimmt wird und anhand der Herzrate die die Belastung beschreibenden Daten bestimmt werden oder dass mindestens ein Zeitintervall in Abhängigkeit der Belastung bestimmt wird.

3. Implantierbares medizinisches System nach Anspruch 1, wobei mindestens ein Drucksensor zur Anordnung in der Pulmonalarterie und/oder der Aorta und/oder den peripheren Arterien und/oder den peripheren Venen und/oder den herznahe Venen und/oder den Herzkammern eingerichtet ist.

4. Implantierbares medizinisches System nach Anspruch 1 oder 3, wobei die diagnostische Größe dadurch bestimmt wird, dass mindestens zwei verschiedene Fitfunktionen an eine Kurve gefittet werden, wobei die Kurve die Abhängigkeit der Atmung von den die Belastung beschreibenden Daten beschreibt oder, eine Fitfunktion an eine Kurve gefittet wird, wobei die Kurve die Abhängigkeit der Atmung von den die Belastung beschreibenden Daten beschreibt.

5. Implantierbares medizinisches System nach Anspruch 4, wobei Fitparameter bestimmt werden und in Abhängigkeit der Fitparameter mindestens eine Aktion gestartet wird.

6. Implantierbares medizinisches System nach Anspruch 1 oder 3 umfassend:
- einen Aktivitätssensor
wobei das System derart eingerichtet ist, dass folgende Schritte ausführbar sind:
- zumindest zeitweiliges Erfassen von Signalen des mindestens einen Aktivitätssensors und Bestimmung von Daten, die eine Belastung beschreiben, durch Auswertung der Signale des mindestens einen Aktivitätssensors,
- Auswertung der Atmung zur Bestimmung einer diagnostischen Größe in Abhängigkeit von den die Belastung beschreibenden Daten.

7. Implantierbares medizinisches System nach Anspruch 6, wobei die die Atmung beschreibenden Daten aus dem durch den mindestens einen Drucksensor erfassten Signal abgeleitet werden.

8. Verfahren zum Monitoring von Atemparametern, wobei
- zumindest zeitweilig Blutdruckwerte erfasst werden,
- durch Auswertung der erfassten Blutdruckwerte Daten bestimmt werden, die eine Belastung beschreiben,
- durch Auswertung der erfassten Blutdruckwerte Daten bestimmt werden, die die Atmung beschreiben,
- eine Abhängigkeit der Atmung von den die Belastung beschreibenden Daten oder eine Abhängigkeit der die Belastung beschreibenden Daten von der Atmung ausgewertet wird zur Bestimmung einer diagnostischen Größe,
**dadurch gekennzeichnet, dass** in Abhängigkeit der Belastung Zeitintervalle einer vorgebbaren Länge bestimmt werden, zumindest einem Teil der Zeitintervalle eine Belastungsstufe zugeordnet wird, und die die Atmung beschreibenden Daten für zumindest einen Teil der Zeitintervalle ausgewertet werden.

9. Verfahren zum Monitoring von Atemparametern nach Anspruch 8, wobei zumindest zeitweilig Signale mindestens eines Aktivitätssensors erfasst und durch Auswertung der Signale des mindestens einen Aktivitätssensors Daten bestimmt werden, die eine Belastung beschreiben.

10. Computerprogramm, das es einer Datenverarbeitungseinrichtung ermöglicht, nachdem es in Speichermittel der Datenverarbeitungseinrichtung geladen worden ist, ein Verfahren zum Monitoring von Atemparametern gemäß einem der Ansprüche 8 oder 9 durchzuführen.

11. Computerlesbares Speichermedium, auf dem ein Programm gespeichert ist, das es einer Datenverarbeitungseinrichtung ermöglicht, nachdem es in Speichermittel der Datenverarbeitungseinrichtung geladen worden ist, ein Verfahren zum Monitoring von Atemparametern gemäß einem der Ansprüche 8 oder 9 durchzuführen.

## Claims

1. An implantable medical system comprising at least
- one pressure sensor,
- one time-registration unit, and
- one data processing unit,
wherein the system is designed such that the following steps can be carried out:
- register blood pressure values, at least temporarily,
- evaluate the registered blood pressure values to determine data that describe a load,
- evaluate the registered blood pressure values to determine data that describe respiration,
**characterized in that**
time intervals of a predefinable length are determined depending on the load, a load stage is ascribed to at least one part of the time intervals, and the data describing the respiration are evaluated for at least one part of the time intervals.

2. The implantable medical system according to claim 1,
wherein
the heart rate is determined on the basis of the blood pressure values, and, on the basis of the heart rate, the data describing the load are determined, or at least one time interval of the load is determined.

3. The implantable medical system according to claim 1,
wherein
at least one pressure sensor is designed to be disposed in the pulmonary artery and/or the aorta and/or the peripheral arteries and/or the peripheral veins and/or the veins close to the heart and/or the ventricles.

4. The implantable medical system according to claim 1 or 3,
wherein
the diagnostic quantity is determined by
fitting at least two different fit functions to a curve, wherein the curve describes the dependence of respiration on the data describing the load, or
a fit function is fitted to a curve, wherein the curve describes the dependence of respiration on the data describing the load.

5. The implantable medical system according to claim 4,
wherein
fit parameters are determined, and at least one action is started depending on the fit parameters.

6. The implantable medical system according to claim 1 or 3, comprising:
- one activity sensor
wherein the system is designed such that the following steps can be carried out:
- register signals, at least temporarily, of the at least one activity sensor, and determine data that describe a load by evaluating the signals of the at least one activity sensor,
- evaluate respiration to determine a diagnostic quantity depending on the data that describe the load.

7. The implantable medical system according to claim 6,
wherein
the data describing respiration are derived from the signal detected by the at least one pressure sensor.

8. A method for monitoring respiratory parameters,
wherein
- blood pressure values are registered, at least temporarily,
- data that describe a load are determined by evaluating the blood pressure values that were registered,
- data that describe respiration are determined by evaluating the blood pressure values that were registered,
- a dependence of respiration on the data describing the load, or a dependence of the data describing the load on respiration is evaluated to determine a diagnostic quantity,
**characterized in that**
time intervals of a predefinable length are determined depending on the load, a load stage is ascribed to at least one part of the time intervals, and the data describing the respiration are evaluated for at least one part of the time intervals.

9. A method for monitoring respiratory parameters according to claim 8,
wherein
- signals from at least one activity sensor are registered, at least temporarily, and data that describe a load are determined by evaluating the signals of the at least one activity sensor.

10. A computer program that enables a data processing device - once said program has been loaded into memory means of the data processing device - to implement a method for monitoring respiratory parameters according to one of the claims 8 or 9.

11. A computer-readable memory medium on which a program is stored that enables a data processing device - once said program has been loaded into memory means of the data processing device - to implement a method for monitoring respiratory parameters according to one of the claims 8 or 9.

## Revendications

1. Système médical implantable comprenant au moins
- un capteur de pression,
- une unité d'enregistrement du temps et
- une unité de traitement de données,
où le système est conçu de telle manière que les étapes suivantes peuvent être exécutées :
- au moins une saisie de valeurs de la tension artérielle en fonction du temps,
- la détermination de données qui décrivent une sollicitation par l'exploitation des valeurs de la tension artérielle,
- la détermination de données qui décrivent la respiration par l'exploitation des valeurs de la tension artérielle,
**caractérisé en ce que**
des intervalles de temps d'une taille pouvant être prédéfinie sont déterminés en fonction de la sollicitation, qu'au moins une partie des intervalles de temps est associée à un degré de sollicitation et que les données décrivant la respiration sont évaluées pour au moins une partie des intervalles de temps.

2. Système médical implantable selon la revendication 1, dans lequel le rythme cardiaque est déterminé à partir des valeurs de la tension artérielle saisies et les données décrivant la sollicitation sont déterminée à l'aide du rythme cardiaque ou qu'au moins un intervalle de temps est déterminé en fonction de la sollicitation.

3. Système médical implantable selon la revendication 1, dans lequel au moins un capteur de pression est conçu pour un agencement dans l'artère pulmonaire, et/ou l'aorte, et/ou les artères périphériques, et/ou les veines périphériques, et/ou les veines proches du coeur, et/ou les ventricules.

4. Système médical implantable selon la revendication 1 ou la revendication 3, dans lequel la grandeur diagnostique est déterminée par le fait qu'au moins deux fonctions d'ajustement différentes sont ajustées à une courbe, où la courbe décrit des données décrivant la dépendance de la respiration avec la sollicitation, ou
une fonction d'ajustement est ajustée à une courbe, où la courbe décrit des données décrivant la dépendance de la respiration avec la sollicitation.

5. Système médical implantable selon la revendication 4, dans lequel des paramètres d'ajustement sont déterminés et au moins une action est démarrée en fonction des paramètres d'ajustement.

6. Système médical implantable selon la revendication 1 ou la revendication 3, comprenant :
- un capteur d'activité,
où le système est conçu de telle manière que les étapes suivantes peuvent être exécutées :
- au moins une saisie de signaux de l'au moins un capteur d'activité en fonction du temps et la détermination de données qui décrivent une sollicitation par l'exploitation des signaux de l'au moins un capteur d'activité,
- l'exploitation de la respiration pour la détermination d'une grandeur diagnostique en fonction des donnés décrivant la sollicitation.

7. Système médical implantable selon la revendication 6, dans lequel les données décrivant la respiration sont déduites à partir du signal saisi par l'au moins un capteur de pression.

8. Procédé de surveillance de paramètres respiratoires, dans lequel
- des valeurs de la tension artérielle sont saisies au moins en fonction du temps,
- des données qui décrivent une sollicitation sont déterminées par l'exploitation des valeurs de la tension artérielle saisies,
- des données qui décrivent la respiration sont déterminées par l'exploitation des valeurs de la tension artérielle saisies,
- des données décrivant une dépendance de la respiration avec la sollicitation ou des données décrivant une dépendance avec la sollicitation sont exploitées pour la détermination d'une grandeur diagnostique,
**caractérisé en ce que**
des intervalles de temps d'une taille pouvant être prédéfinie sont déterminés en fonction de la sollicitation, qu'au moins une partie des intervalles de temps est associée à un degré de sollicitation et les données décrivant la respiration sont évaluées pour au moins une partie des intervalles de temps.

9. Procédé de surveillance de paramètres respiratoires selon la revendication 8, dans lequel des signaux d'au moins un capteur d'activité sont détectés au moins en fonction du temps et des données qui décrivent une sollicitation sont déterminées par l'exploitation des signaux de l'au moins un capteur d'activité.

10. Programme informatique qui permet à un dispositif de traitement de données, lorsqu'il a été chargé dans un support de stockage du dispositif de traitement de données, d'exécuter un procédé de surveillance de paramètres respiratoires selon l'une des revendications 8 ou 9.

11. Support de stockage lisible par ordinateur sur lequel est stocké un programme qui permet à un dispositif de traitement de données, lorsqu'il a été chargé dans un support de stockage du dispositif de traitement de données, d'exécuter un procédé de surveillance de paramètres respiratoires selon l'une des revendications 8 ou 9.
